# EUROPEAN PATENT SPECIFICATION

(11) **EP 4 344 628 B1**
(45) Date of publication and mention of the grant of the patent: **11.11.2026**
(21) Application number: 21948571.1
(22) Date of filing: 11.11.2021
(51) Int. Cl.: A61B 5/145, A61B 5/155, A61B 5/15, A61M 5/172, A61M 5/142

(54) **APPLICATOR WITH NEEDLE FOR INSERTING TRANSCUTANEOUS SENSOR**
APPLIKATOR MIT NADEL ZUM EINSETZEN EINES TRANSKUTANEN SENSORS
APPLICATEUR AVEC AIGUILLE POUR L'INSERTION D'UN CAPTEUR TRANSCUTANÉ

(30) Priority: 29.06.2021 KR 20210084617
(43) Date of publication of application: 03.04.2024
(73) Proprietor: i-Sens, Inc., Seoul 06646 (KR)
(72) Inventor: CHOI, Hyun Ho, Seoul 06646 (KR); RYU, Goang Yel, Seoul 06646 (KR); WANG, Ji Hoon, Seoul 06646 (KR)
(74) Representative: BCKIP Part mbB
(86) International application number: PCT/KR2021/016418
(87) International publication number: WO 2023/277271

(56) References cited:
- JP-A- 2016 128 031
- KR-A- 20210 016 312
- KR-A- 20210 018 631
- US-A1- 2004 133 164
- US-A1- 2010 249 565
- US-A1- 2017 245 798
- US-A1- 2021 052 224

## Description

### TECHNICAL FIELD

The present invention relates to an applicator comprising a needle for inserting a transdermal sensor, particularly comprising a needle for inserting a sensor which is inserted into the skin of a user and measures biometric information into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for biometric information measurement that is attached to the body can be used to manage blood glucose levels in diabetic patients.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of glucose. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a sensor unit that includes a transcutaneous sensor that is inserted into the skin of a user and measures blood glucose levels in the body, a base unit that is electrically connected to the sensor unit and generates a signal from biometric information measured by the sensor, and a terminal that receives and outputs a signal transmitted from the base unit.

An applicator is used to insert the sensor into the body. The applicator includes a needle that is inserted into the skin of a user along with a sensor to insert the sensor into the skin of a user. The needle is removed from the skin of a user after the sensor is inserted into the skin of a user. The needle is generally coupled to a carrier that is provided to be movable in the applicator and moves by the carrier.

Conventionally, needles were manufactured by coupling a separate injection molded product that could be coupled to a carrier to a needle body that was inserted into the skin of a user. However, these conventional needles have a problem in which the manufacturing cost is high and the manufacturing time is long.

US 2021/052224 A1 discloses an example of an applicator of an on-skin sensor assembly. The applicator includes an applicator housing, a needle carrier assembly comprising an insertion element configured to insert a sensor of the on-skin sensor assembly into the skin of the host, a holder releasably coupled to the needle carrier assembly and configured to guide the on-skin sensor assembly while coupled to the needle carrier assembly, and a drive assembly configured to drive the insertion element from a proximal starting position to a distal insertion position, and from the distal insertion position to a proximal retraction position.

US 2017/245798 A1 discloses a sensor which measures biological information and an insertion needle. The sensor includes a detector which detects a body fluid component of the patient used to measure the biological information, and a signal processor which is attached to the device body and which processes a signal including the biological information. The sensor and the insertion needle are moved in a distal direction so that the detector and a portion of the insertion needle protrude distally beyond a distal end portion of a device body. Afterwards, the insertion needle is separated from the sensor by moving the insertion needle relative to the sensor toward a proximal direction.

### SUMMARY OF THE INVENTION

### Technical Problem

The present invention is developed in consideration of the above-mentioned points, and the purpose is to provide a needle and needle assembly for inserting a transcutaneous sensor that has a simple structure, low manufacturing cost, and is easier to couple with the sensor.

### Solution to Problem

To accomplish the above-described purposes, the present invention provides an applicator in accordance with claim 1.

A needle included in an applicator for inserting a sensor for measuring biometric information into skin of a user and configured to be insertable into the skin of the user with the sensor according to the present invention comprises: a needle body having a first channel accommodating the sensor and to be insertable into the skin of the user; and a needle head with a thickness greater than the needle body, wherein the needle body and the needle head are formed as one integrated piece by being press-processed from same base material.

The needle, according to the present invention, comprises a needle neck formed as one integrated piece with the needle body and the needle head to connect the needle body and the needle head, and having a shape in which a thickness of the needle neck gradually decreases from the needle head toward the needle body.

The needle head includes a second channel having a width wider than the first channel, and in the needle neck, a third channel of which width gradually decreases from the second channel to the first channel is provided to be connected to the first channel and the second channel, respectively.

According to an embodiment, the needle body comprises a needle body base having a needle tip at an end of the needle body base, the needle head comprises a needle head base disposed on a same plane as the needle body base, and the needle neck is formed with a shape of which width gradually decreases from the needle head base toward the needle body base and includes a needle neck base disposed on a same plane as the needle body base and the needle head base to connect the needle body base and the needle head base.

According to an embodiment, the needle body comprises a first needle body wing and a second needle body wing bent at both sides of the needle body base to form the first channel with the needle body base, the needle head comprises a first needle head wing and a second needle head wing bent at both sides of the needle head base to form a second channel with the needle head base, and the needle neck comprises a first needle neck wing bent at one side of the needle neck base and connecting the first needle body wing and the first needle head wing, and a second needle neck wing bent at an other side of the needle neck base and connecting the second needle body wing and the second needle head wing, and the first needle neck wing and the second needle neck wing with the needle neck base form a third channel connected to the first channel and the second channel, respectively.

The needle according to an embodiment of the present invention may comprise a needle tail extended from an end of the needle body base to be disposed on a same plane as the needle body base and having a width narrower than the needle body base.

According to the invention, the needle head has a locking portion configured to be engageable with a carrier included in the applicator.

The locking portion is formed as one integrated piece with the needle head and includes a hook portion protruding from the needle head.

A carrier of the applicator may have a clamp portion engaged with the needle head, wherein the carrier is movably mounted to an applicator body of the applicator and coupled with the needle to move with the needle.

The carrier has an insertion groove into which the needle head is inserted, and the clamp portion is disposed in the insertion groove.

The carrier comprises a carrier body having the clamp portion and a pair of locking arms elastically deformably connected to the carrier body to be detachably engageable with both edges of the sensor housing to which the sensor is mounted.

### Advantageous Effects of Invention

The needle has a simple structure, is easy to manufacture, and has a low manufacturing cost because the needle body and the needle head are press-processed from the same base material to form one piece.

Additionally, the needle can be mass-produced by press processing a single base material.

Additionally, the needle can be more simply assembled with the sensor or the carrier of the applicator.

### BRIEF DESCRIPTION OF THE DRAWINGS

FIG. 1 is a cross-sectional view showing an applicator according to an embodiment of the invention, including a needle assembly.
FIG. 2 shows an example of a body attachable unit attached to the skin of a user by an applicator according to an embodiment of the invention, including a needle assembly.

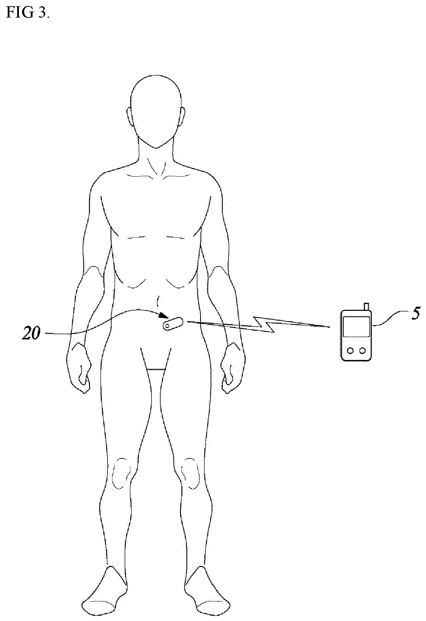

FIG. 3 shows a body attachable unit attached to the skin of a user.

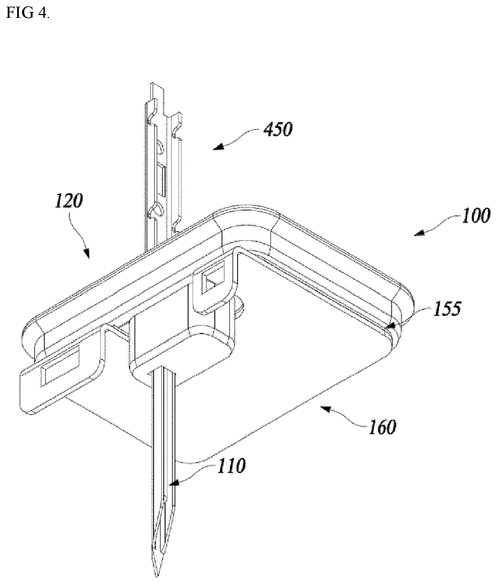

FIG. 4 shows a protective sheet attached to the adhesive layer of a sensor unit.

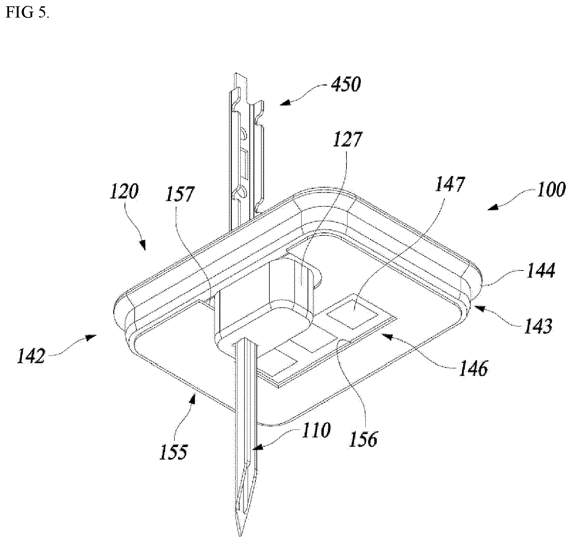

FIG. 5 shows an appearance after the protective sheet is separated from the adhesive layer of the sensor unit.
FIG. 6 shows a sensor unit and a base unit before they are coupled.

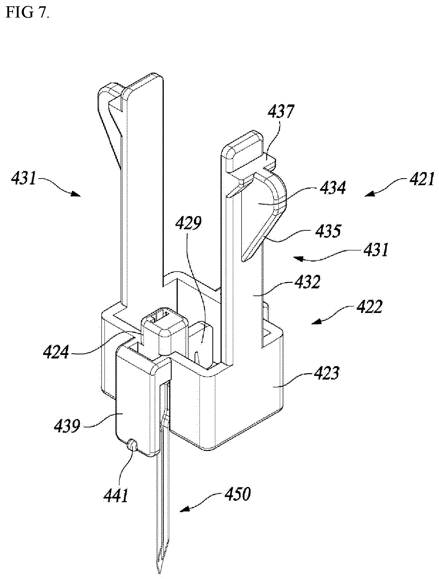

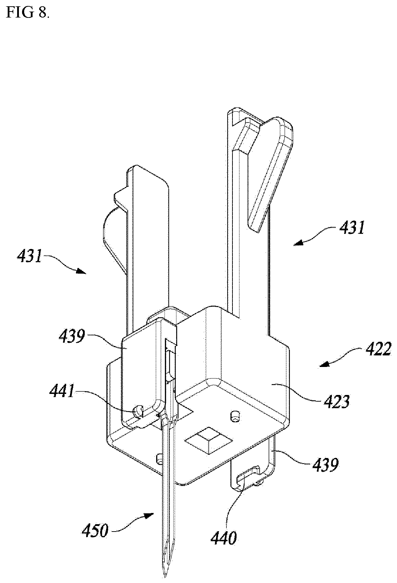

FIG. 7 and 8 are perspective views showing a needle assembly for an applicator according to an embodiment of the present invention.
FIG. 9 and 10 are perspective views showing a needle not covered by the claimed invention.
FIG. 11 is a front view showing the needle of FIGS. 9 and 10.
FIG. 12 is a side view showing the needle of FIGS. 9 and 10.
FIG. 13 shows an appearance before a plurality of needles which are press-processed from one base material are separated individually.
FIG. 14 is a cross-sectional view showing the carrier and the needle, which is not covered by the present invention, before being coupled.
FIG. 15 is a cross-sectional view showing the carrier and a needle, which is not covered by the present invention, coupled.
FIGS. 16 to 18 show a process in which the body attachable unit is attached to the skin of a user by an applicator.
FIGS. 19 and 20 are perspective views showing a needle according to another example not covered by the claimed invention.
FIG. 21 is a front view showing a needle according to another example not covered by the claimed invention.
FIG. 22 is a side view showing a needle according to another example not covered by the claimed invention.
FIG. 23 is a perspective view showing a needle according to another example not covered by the claimed invention.
FIGS. 24 and 25 are perspective views showing a needle for an applicator according to an embodiment of the present invention.
FIG. 26 is a front view showing a needle for an applicator according to another embodiment of the present invention.
FIG. 27 is a side view showing a needle for an applicator according to another embodiment of the present invention.
FIG. 28 is a perspective view showing a needle for an applicator according to another embodiment of the present invention.
FIG. 29 is a perspective view showing a needle for an applicator according to another embodiment of the present invention.

### DETAILED DESCRIPTION OF EMBODIMENTS

Hereinafter, a needle and needle assembly for inserting a transcutaneous sensor according to the present invention will be described in detail with reference to the drawings.

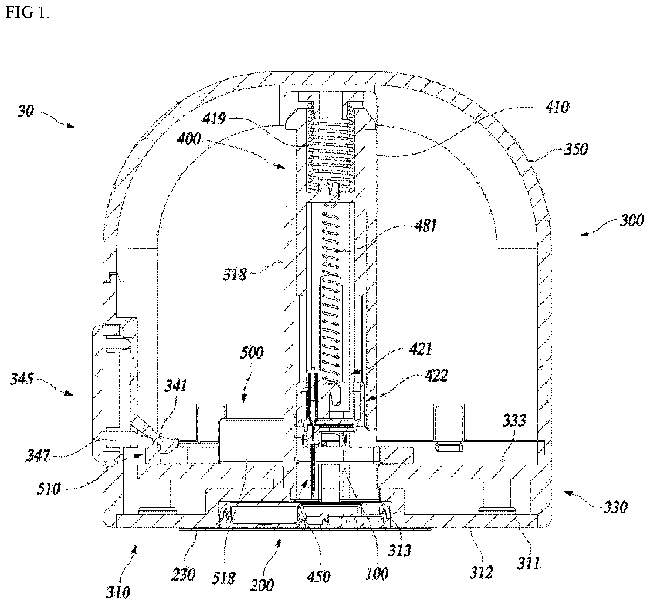

FIG. 1 is a cross-sectional view showing an applicator including a needle assembly.

The needle assembly (421) according to an embodiment of the present invention is provided on the applicator (30) and may insert the sensor (110) of the sensor unit (100) into the skin of a user. The applicator (30) may be provided to the user with the sensor unit (100), which has the sensor (110), and the base unit (200), which is combined with the sensor unit (100) and constitutes the body attachable unit (20), separately mounted. The sensor unit (100) and the base unit (200) can be automatically assembled and attached to the skin of a user through the action of the applicator (30).

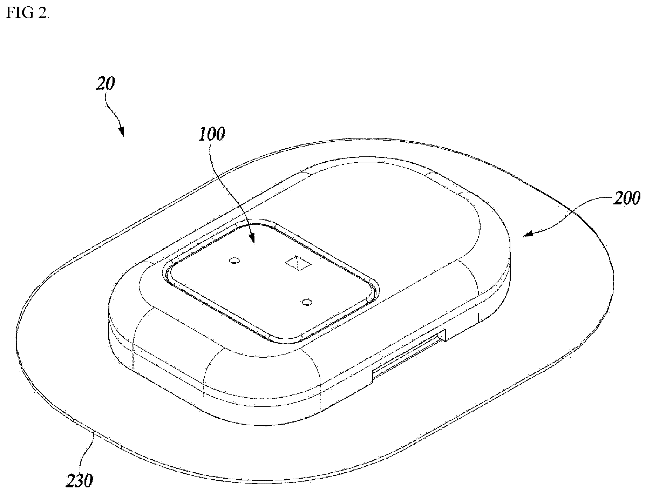

As shown in FIG. 2, the body attachable unit (20) can measure biometric information by being attached to the skin of a user by the applicator (30) and wirelessly transmit the measured data to the external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may periodically measure the blood glucose level of a user and transmit the blood glucose measurement information to the external terminal (5).

The sensor unit (100) includes a sensor (110) inserted into the skin of a user, a sensor unit housing (120) to which the sensor (110) is coupled, a sensor unit-electrical contact portion (146) coupled to the sensor unit housing (120) and electrically connected to the sensor (110), an adhesive layer (155) provided on the sensor unit housing (120) so as to be attached to the base unit (200), and protective sheet (160) covering the adhesive layer (155).

A portion of the sensor (110) is disposed inside the sensor housing (120) and is electrically connected to the sensor unit-electrical contact portion (146). The sensor (110) includes an insertion portion (116) that protrudes from the sensor housing (120) and is inserted into the skin of a user.

In the sensor unit housing (120), a housing opening into which a part of the sensor (110) and a needle (450) which is for inserting the sensor (110) into the skin of a user are inserted is formed to penetrate the sensor unit housing (120) in the thickness direction. The sensor unit housing (120) includes a housing body (142) and a boss (127) protruding from one side of the housing body (142). The housing opening is formed to penetrate the housing body (142) and the boss (127). The housing body (142) is shaped to fit into the base unit recess (213) of the base unit (200). The housing body (142) includes a body portion (143) provided with a boss (127) and a cover portion (144) that is wider than the body portion (143). The sensor unit housing (120) is not limited to the configuration shown, and may be changed to various other configurations on which the sensor (110) may be mounted and coupled to the base unit (200).

The sensor unit-electrical contact portion (146) is disposed in the sensor unit housing (120) to be electrically connected to the sensor (110). The sensor unit-electrical contact portion (146) may include a plurality of terminal portions (147) for transmitting electrical signals. A portion of the sensor unit-electrical contact portion (146) is exposed to the surface of the sensor unit housing (120), thereby electrically connecting the sensor (110) and the base unit-electrical contact portion (225) of the base unit (200).

Adhesion layer (155) is disposed on the surface of sensor unit housing (120). The adhesive layer (155) has adhesive properties on both sides. One side of the adhesive layer (155) is adhered to the surface of the sensor unit housing (120), and the other side of the adhesive layer (155) is covered with a protective sheet (160). The adhesive layer (155) may be adhered to the base unit (200) after the protective sheet (160) is separated. An adhesive layer hole (156) is formed in the middle portion of the adhesive layer (155) to penetrate the adhesive layer (155) in the thickness direction. The sensor unit-electrical contact portion (146) may contact the base unit-electrical contact portion (225) of the base unit (200) through the adhesive layer hole (156). Therefore, the adhesive layer (155) seals between the sensor unit-electrical contact portion (146) and the base unit-electrical contact portion (225) to prevent moisture or foreign substances from entering the electrical connection portion between the sensor unit (100) and the base unit (200). An adhesive layer opening (157) is formed on one side of the adhesive layer (155) to penetrate the adhesive layer (155) in the thickness direction. The adhesive layer (155) is attached to the housing base (121) such that the sensor unit-electrical contact portion (146) is located inside the adhesive layer hole (156) and the boss (127) is inserted into the adhesive layer opening (157).

The protective sheet (160) covers and protects the adhesive layer (155). The protective sheet (160) is made of a material that is detachably attached to the adhesive layer (155). If the adhesive layer (155) is left exposed to the air for a long time, the adhesiveness of the adhesive layer (155) may deteriorate. The protective sheet (160) covers the adhesive layer (155) to prevent the problem of poor adhesion of the adhesive layer (155), and facilitates handling of the sensor unit (100) by an operator in the process of manufacturing the sensor unit (100) or assembling the sensor unit (100) to the applicator (30).

The sensor unit (100) is mounted on the applicator (30) in a state in which the protective sheet (160) is attached to the adhesive layer (155). The protective sheet (160) may be separated from the adhesive layer (155) by the removing unit (500) of the applicator (30) before the sensor (110) is inserted into the skin of a user. The sensor unit (100) is coupled to the base unit (200) by moving toward the base unit (200) in a state in which the protective sheet (160) is separated.

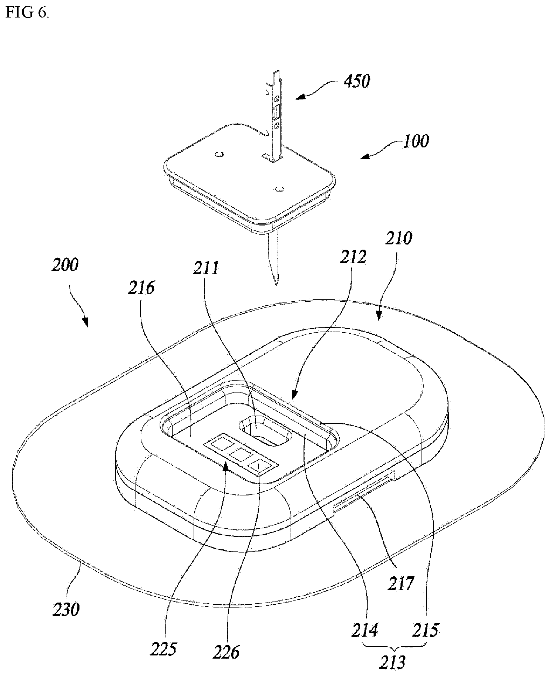

As shown in FIG. 6, the base unit (200) includes a base unit housing (210) to which the sensor unit (100) is coupled, and electronic components installed inside the base unit housing (210). The electronic components include a base unit-electrical contact portion (225) that contacts the sensor unit-electrical contact portion (146) of the sensor unit (100), a circuit board, a battery, a processor chip for processing signals, and a communication chip for wireless communication with the external terminal (5).

The base unit housing (210) is provided with an insertion hole (211) through which the sensor (110) and the needle (450) can pass, and a mounting portion (212) to which the sensor unit housing (120) is coupled. The mounting portion (212) includes a base unit recess (213) and a contact surface (216) provided inside the base unit recess (213). The insertion hole (211) is shaped so that the boss (127) of the sensor unit (100) can be fitted and is disposed inside the base unit recess (213). The contact surface (216) may be flat so that the adhesive layer (155) of the sensor unit (100) can be stably adhered. The base unit recess (213) includes a first recess (214) connected to the insertion hole (211), and a second recess (215) that is located farther from the insertion hole (211) than the first recess (214) and is connected to the first recess (214). The second recess (215) is wider than the first recess (214). The first recess (214) can be made in a shape corresponding to the body portion (143) of the sensor unit housing (120), and the second recess (215) can be made in a shape corresponding to the cover portion (144) of the sensor unit housing (120). Therefore, the sensor unit housing (120) can be fitted into the base unit recess (213) and maintain a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). A portion of a locking hook for detachably fixing the base unit (200) to the applicator (30) may be inserted into the housing groove (217).

The base unit-electrical contact portion (225) is arranged on the mounting portion (212) and contacts the sensor unit-electrical contact portion (146) of sensor unit (100) when sensor unit (100) is coupled to the base unit (200). The base unit-electrical contact portion (225) is installed in the base unit housing (210) so that a portion is exposed into the base unit recess (213). The base unit-electrical contact portion (225) may include a plurality of terminal portions (226) for transmitting electrical signals. The terminal portion (226) may electrically connect the sensor unit-electrical contact portion (146) and the circuit board (223) by contacting the terminal portion (147) of the sensor unit (100).

In order to electrically connect the sensor (110) and the base unit (200), the specific configuration, number, and location of the base unit-electrical contacts portion provided in the base unit (200) may be changed in various ways. Additionally, the configuration for electrically connecting the sensor (110) and the base unit (200) is not limited to that shown and may vary in various ways.

An adhesive portion (230) is provided on the surface of the base unit housing (210). The adhesive portion (230) may be attached to the surface of the lower housing (219) to adhere the base unit housing (210) to the skin of a user. The adhesive portion (230) may be covered with a protective sheet and protected. The protective sheet covering the adhesive portion (230) may be removed in the process of attaching the base unit (200) to the skin of a user.

The sensor unit (100) and the base unit (200) are installed on the applicator (30) in a separated state, and in the process in which the applicator (30) is operated to insert the sensor (110) into the skin of user, they are coupled to each other and then form a body attachable unit. The sensor unit (100) may be mounted on the applicator (30) in a state in which the needle (450) is coupled, and moved toward the base unit (200) with the needle (450) to be coupled to the base unit (200). After the sensor unit (100) is coupled to the base unit (200), the needle (450) is separated from the sensor unit (100), and only the body attachable unit (20) remains on the skin of a user.

The applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The sensor unit (100) and the base unit (200) are spaced apart from each other and are respectively mounted on the applicator (30). The applicator (30) is separated from the body attachable unit (20) after attaching the body attachable unit (20) to the skin of a user, and the body attachable unit (20) remains attached to the skin of a user by the adhesive portion (230).

The applicator (30) includes an applicator body (300) to which the base unit (200) is detachably coupled, an insertion unit (400) that moves the sensor unit (100) to insert the sensor (110) into the skin of a user, and removing unit (500) for removing the protective sheet (160) of the sensor unit (100). The insertion unit (400) may move the sensor unit (100) from a first position spaced apart from the base unit (200) by a preset distance to a second position coupled with the base unit (200). The sensor unit (100) and the base unit (200) are coupled at the second position to form the body attachable unit (20).

The applicator body (300) includes a base frame (310) on which the insertion unit (400) is installed, a middle frame (330) disposed on the base frame (310) and on which the removing unit (500) is installed, and a top case (350) that is coupled to and covers the upper part of the middle frame (330).

The base frame (310) includes a frame base portion (311) having a bottom portion (312) that can be in contact with the skin of a user, and a column portion (318) which protrudes from the frame base portion (311) and accommodates the sensor unit (100) and the insertion unit (400). The bottom portion (312) of the frame base portion (311) is provided with a recess (313) into which the base unit (200) is mounted. The base unit (200) is detachably coupled to the recess (313) so that the adhesive portion (230) can face the skin of a user and placed in a second position spaced apart from the sensor unit (100).

The middle frame (330) includes a stage (333) disposed on the frame base portion (311). The stage (333) supports the moving member (510) of the removing unit (500) to move linearly. An opening into which the column portion (318) is inserted is formed in the middle of the stage (333), so that the column portion (318) can protrude above the stage (333). The middle frame (330) includes a stopper (341) that engages the moving member (510) to restrict the movement of the moving member (510), and an operating member (345) capable of operating the insertion unit (400) and the removing unit (500). The stopper (341) may be released by the operating member (345) so that the binding force on the moving member (510) may be removed. The operating member (345) is pivotally coupled to the middle frame (330) and has a pressing protrusion (347) capable of pressing the stopper (341). When a user presses the operating member (345), the pressing protrusion (347) of the operating member (345) may press the stopper (341). At this time, the stopper (341) may be elastically deformed and disengaged from the moving member (510).

The top case (350) is coupled with the middle frame (330) and covers the middle frame (330) and the upper portions of the base frame (310).

The insertion unit (400) is installed on the applicator body (300) to associate with the moving member (510) of the removing unit (500) to move the sensor unit (100) from the first position to the second position, and the sensor (110) can be inserted into the skin of a user. The insertion unit (400) includes a plunger (410) installed movably on the middle frame (330) and a needle assembly (421) having a needle (450) that can be moved with the plunger (410) and inserted into the skin of a user.

The plunger (410) is installed inside the column portion (318) to be movable from the first position to the second position. The plunger (410) is fixed in the first position by partially engaging the moving member (510), and can move to the second position when the engagement is released from the moving member (510). The plunger (410) moves by receiving a moving force from the elastic member (419). The elastic member (419) may apply elastic force to the plunger (410) in a direction moving from the first position to the second position.

The needle assembly (421) includes the carrier (422) to which the sensor unit (100) is detachably coupled, and a needle (450) coupled to the carrier (422) penetrating the sensor unit (100). In a state in which the needle (450) is coupled to the sensor unit (100), the needle (450) advance from the first position to the second position together with the sensor unit (100) to be inserted into the skin of a user, and then may retreat with the carrier (422) in the second position and be separated from the skin of a user and the sensor unit (100).

The carrier (422) is coupled to the plunger (410) to enable relative movement. A portion of the carrier (422) engages the plunger (410) so as to move with the plunger (410) from the first position to the second position. Then, the carrier (422) is disengaged from the plunger (410) in the second position and may retreat in a direction away from the sensor unit (100).

The carrier (422) includes a carrier body (423) to which the needle (450) is coupled, an elastically deformable carrier wing (431) extending from the carrier body (423), and a locking arm (439) connected to the side of the carrier body (423).

The carrier body (423) includes a boss (424) with an insertion groove (425) formed therein. A portion of the needle (450) may be inserted into the insertion groove (425). A clamp portion (427) for fixing the needle (450) is provided in the insertion groove (425). The clamp portion (427) includes a hook (428) that can engage with the needle (450). A fixing portion (429) to which an elastic member (481) is coupled is provided on one side of the carrier body (423). The carrier (422) can be moved relative to the plunger (410) by the elastic member (481). One end of the elastic member (481) is coupled to the plunger (410) and the other end is coupled to the fixing portion (429) of the carrier (422). The elastic member (481) is in the form of a tension spring and applies elastic force to the carrier (422) in a direction away from the base unit (200).

The carrier wing (431) is connected to the carrier body (423) in a shape that extends from the carrier body (423) in the moving direction of the carrier (422). The carrier wing (431) includes a wing body (432) elastically deformably connected to the carrier body (423), and a trigger (434) and a latch portion (437) protruding from the wing body 432.

The trigger (434) is provided on one side of the wing body (432) to protrude outward from the wing body (432). While the carrier (422) moves from the first position to the second position with the plunger (410), the trigger (434) may contact one side of the column portion (318) and be elastically deformed.

The latch portion (437) protrudes outward from the wing body (432). The latch portion (437) may be engaged with one side of the plunger (410). In a state in which the latch portion (437) is engaged with the plunger (410), the carrier (422) may maintain a state in which the elastic member (481) is elastically deformed, and may not move in a direction in which the elastic force of the elastic member (481) acts.

The locking arm (439) is provided on the side of the carrier body (423) to be elastically deformable. A pair of locking arms (439) are arranged to face each other on both sides of the carrier body (423) and detachably fix the sensor unit housing (120) of the sensor unit (100). The locking arm (439) is provided with a locking protrusion (440) engaged with the sensor unit housing (120) and a protrusion (441) in contact with an inner wall (323) of the column portion (318). When the carrier (422) and the sensor unit (100) are located in the first position, as the protrusion (441) contacts the inner wall (323) of the column portion (318), the locking arm (439) is biased in a direction in which it engages with the sensor unit housing (120). Accordingly, the sensor unit (100) can remain stably coupled to the carrier (422) in the first position.

In addition to the configuration shown, the carrier (422) can be changed to various other configurations in which it is coupled to the needle (450) and installed to be relatively movable on the plunger (410). For example, the drawing shows a pair of carrier wings (431) being symmetrically provided on both sides of the carrier body (423), but the number and shape of the carrier wings (431) can be changed in various ways. Additionally, the coupling method of the carrier (422) and the needle (450) and the coupling method of the carrier (422) and the sensor unit (100) may also be changed in various ways.

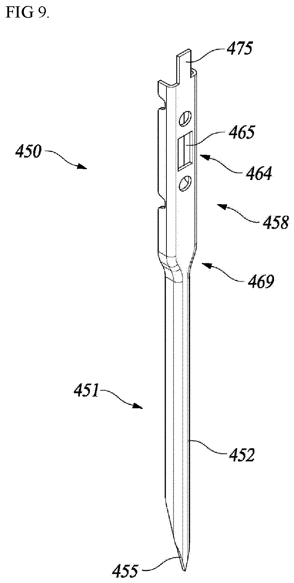

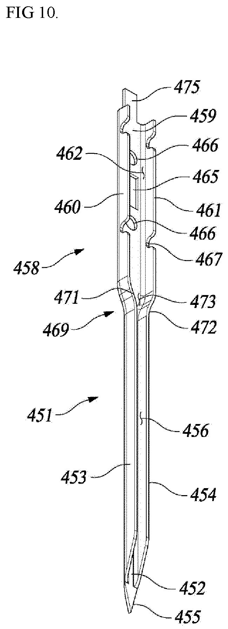

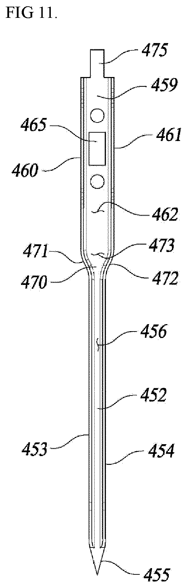

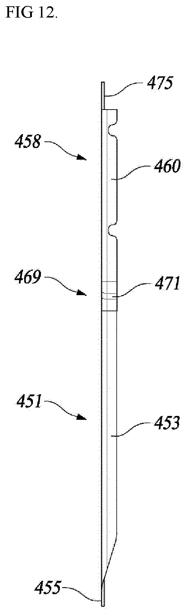

Referring to FIGS. 9 to 15, the needle (450) is fixed to the carrier (422) and can move from the first position to the second position while coupled to the sensor unit (100). The needle (450) has a sharp tip so as to pierce the skin of a user and be smoothly inserted into the skin of a user. When the sensor unit (100) moves to the second position, the needle (450) penetrates the skin of a user before the sensor (110) and allows the sensor (110) to be stably inserted into the skin. The needle (450) is separated from the skin of a user after the sensor (110) is inserted into the skin of a user.

The needle (450) includes a needle body (451) inserted into the skin of a user, a needle head (458) coupled to the carrier (422), a needle neck (469) connecting the needle body (451) and the needle head (458) and a needle tail (475) extending from the end of the needle head (458). The needle (450) may be formed as one piece by press-processing the needle body (451), the needle head (458), the needle neck (469) and the needle tail (475) from the same base material.

The needle body (451) includes a needle body base (452) equipped with a needle tip (455) at the end, and a first needle body wing (453) and a second needle body wing (454) disposed on both sides of the needle body base (452). The first needle body wing (453) and the second needle body wing (454) are bent at both sides of the needle body base (452) and are arranged to face each other. The needle body base (452), the first needle body wing (453), and the second needle body wing (454) form the first channel (456). The insertion portion (116) of the sensor (110) may be inserted into the first channel (456). The needle (450) is coupled to the sensor unit (100) so that the insertion portion (116) is inserted into the first channel (456).

The needle head (458) includes a needle head base (459) disposed on the same plane as the needle body base (452), and a first needle head wing (460) and a second needle head wing (461) disposed on both sides of the needle head base (459). The first needle head wing (460) and the second needle head wing (461) are bent at both sides of the needle head base (459) and are arranged to face each other. The needle head base (459), the first needle head wing (460), and the second needle head wing (461) form the second channel (462). The width of the second channel (462) is larger than the width of the first channel (456).

The needle head (458) is provided with a locking portion (464) that engages the clamp portion (427) of the carrier (422). The locking portion (464) includes a needle hole (465) into which the hook (428) of the clamp portion (427) can be inserted. The needle hole (465) is provided in a form that penetrates the needle head base (459) in the thickness direction. The needle (450) may be fixed to the carrier (422) in such a way that the needle head (458) is inserted into the insertion groove (425) of the carrier (422) and the hook (428) is inserted into the needle hole (465).

In addition, the needle head (458) is provided with a through hole (466) and a groove portion (467). The through hole (466) is formed to penetrate the needle head base (459) in the thickness direction, and the groove portion (467) is formed in the first needle head wing (460) and the second needle head wing (461) respectively. The through hole (466) and the groove portion (467) may be used to reduce the weight of the needle (450) and prevent deformation of the needle head (458) in the process of forming the needle head (458) from the base material (480). Additionally, the through hole (466) and the groove portion (467) may be used for coupling with the carrier (422).

The needle neck (469) has a shape whose thickness gradually decreases from the needle head (458) toward the needle body (451) and connects the needle body (451) and the needle head (458). The needle neck (469) includes a needle neck base (470) disposed on the same plane as the needle body base (452), a first needle neck wing (471) and a second needle neck wing (472) disposed on both sides of the needle neck base (470). The first needle neck wing (471) and the second needle neck wing (472) are bent at both sides of the needle neck base (470) and are arranged to face each other. The needle neck base (470) connects the needle body base (452) and the needle head base (459), and the first needle neck wing (471) connects the first needle body wing (453) and the first needle head wing (460). And the second needle neck wing (472) connects the second needle body wing (454) and the second needle head wing (461). The needle neck base (470), the first needle neck wing (471), and the second needle neck wing (472) form the third channel (473). The third channel (473) has a shape whose width gradually decreases from the second channel (462) toward the first channel (456), and is connected to the first channel (456) and the second channel (462), respectively.

In the process of coupling the sensor (110) and the needle (450), the sensor (110) and the needle (450) are coupled by inserting the insertion portion (116) of the sensor (110) into the first channel (456) through the second channel (462) and the third channel (473) sequentially. In this way, since the needle (450) has a form in which the insertion portion (116) of the sensor (110) may be inserted into the first channel (456) through the second channel (462) and the third channel (473) which have relatively larger widths, a problem in which the sensor (110) is damaged or deformed when the sensor (110) and the needle (450) are coupled may be reduced.

The needle tail (475) extends from the end of the needle head base (459) to be disposed on the same plane as the needle head base (459). The width of the needle tail (475) is smaller than the width of the needle head base (459).

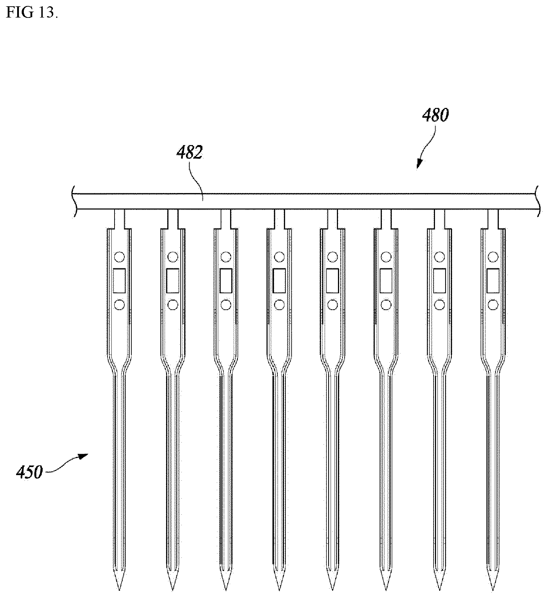

As shown in FIG. 13, needles (450) can be mass-produced through press-processing of a single base material (480). In the process of manufacturing the needle (450) by press-processing the base material (480), the needle (450) may be input into a subsequent process in a state in which it is connected to the strap (482) remaining after the needle (450) is formed. For example, the plurality of needles (450) may be input into the cutting process in a state in which each needle tail (475) is connected to the strap (482) and spaced apart at regular intervals. In the cutting process, the needles (450) may be cut so that the needle tails (475) are separated from the strap (482) and may be separated individually.

In this way, the needle (450) having a shape including the needle tail (475) is advantageous in manufacturing. When the needle (450) is manufactured in a shape without the needle tail (475) by press-processing the base material (480), the needle head (458) is likely to be damaged during the cutting process. Therefore, the needle (450) without the needle tail (475) has a high defective rate during the manufacturing process, but the needle (450) with the needle tail (475) has a low defective rate during the manufacturing process.

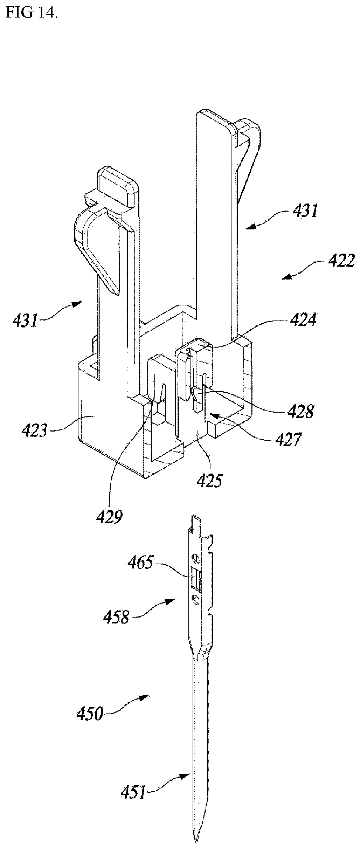

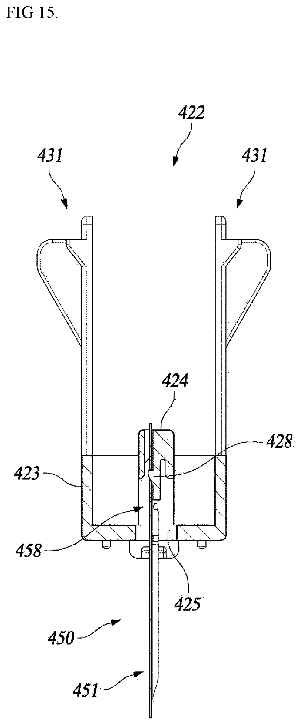

As shown in FIGS. 14 and 15, the needle (450) can be simply assembled to the carrier (422) by simply inserting the needle head (458) into the insertion groove (425) of the carrier (422). When the needle head (458) is inserted into the insertion groove (425) to a certain depth, the hook (428) of the carrier (422) is inserted into the needle hole (465) of the needle head (458), so that the needle (450) can be firmly fixed to the carrier (422).

The needle (450) according to the present invention is formed as one piece by press-processing the needle body (451) and the needle head (458) from the same base material (480), and thus has a simple structure, is easy to manufacture, and has a low manufacturing cost. Additionally, the needle (450) according to the present invention can be more simply assembled with the sensor (110) and the carrier (422).

Referring again to FIG. 1, the removing unit (500) is installed on the applicator body (300) to separate the protective sheet (160) of the sensor unit (100) from the adhesive layer (155) before the sensor unit (100) reaches the second position and is coupled to the base unit (200). The removing unit (500) includes a moving member (510) installed movably on the middle frame (330) to be coupled to the protective sheet (160). The moving member (510) is arranged to move linearly on the stage (333) of the middle frame (330). The moving member (510) includes a support portion (518) for supporting the plunger (410).

The moving member (510) is installed to move in a direction that is approximately perpendicular to the direction in which the sensor unit (100) moves from the first position to the second position. The moving member (510) may pull the protective sheet (160) to be separated from the adhesive layer (155) by moving while coupled with the protective sheet (160). Specifically, in a state in which the protective sheet (160) covers the adhesive layer (155), the moving member (510) can move from a third position where the protective sheet is coupled to a fourth position where the protective sheet (160) is removed from the adhesive layer. The moving member (510) fixes the plunger (410) at the first position by coming into contact with the plunger (410) located in the first position at the third position, and when the moving member moves to the fourth position, it deviates from the plunger (410) so that the plunger 410 can move to the second position. The moving member (510) can move by receiving elastic force from an elastic member installed on the applicator body (300).

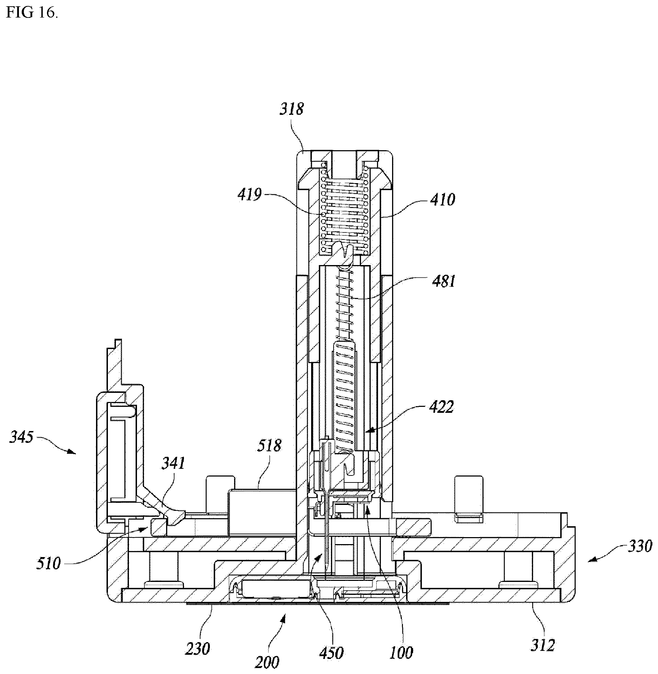

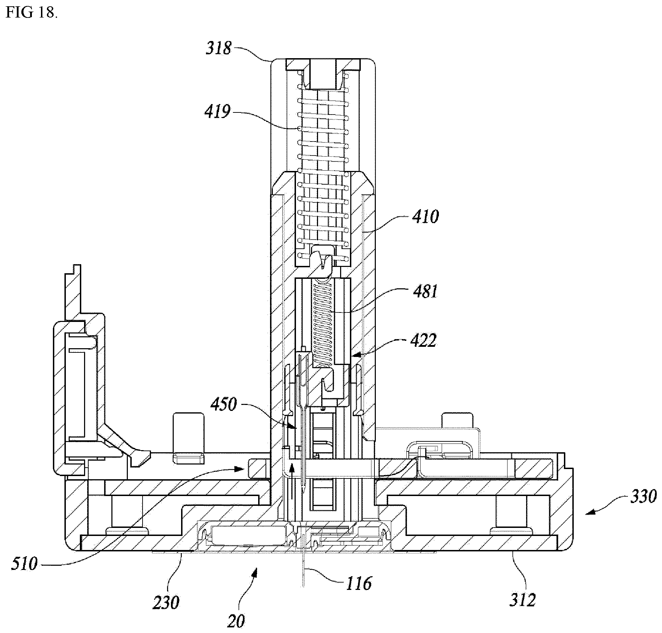

Hereinafter, with reference to FIGS. 16 to 18, a method of attaching the body attachable unit (20) to the skin of a user using the applicator (30) will be described.

Referring to FIG. 16, first, the bottom portion (312) of the applicator (30) is placed on the skin of a user so that the base unit (200) is attached to the skin of a user by the adhesive portion (230). Before the operating member (345) is manipulated, the sensor unit (100) coupled to the carrier (422) is positioned in the first position in a state in which the protective sheet (160) covers the adhesive layer (155). And the moving member (510) is located in the third position engaging with the stopper (341).

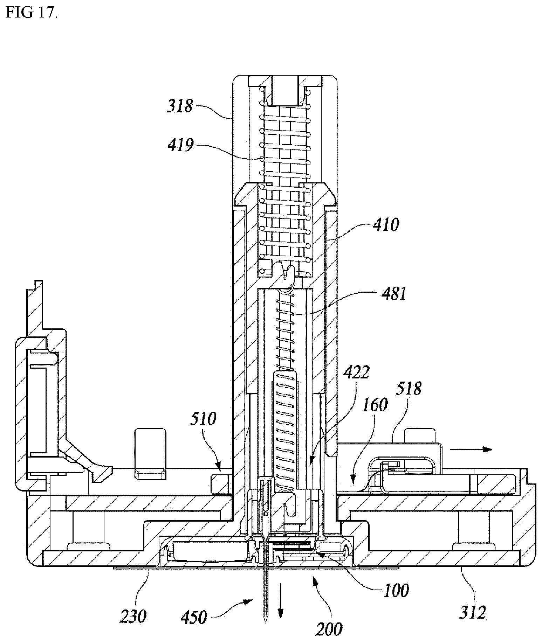

Thereafter, when the operating member (345) is pressed, the moving member (510) is disengaged from the stopper (341) and the moving member (510) is moved to the fourth position by the elastic force of the elastic member. As shown in FIG. 17, as the moving member (510) moves to the fourth position, the protective sheet (160) is pulled by the moving member (510) and separated from the adhesive layer (155). Then, the plunger (410) moves away from the moving member (510) and moves to the second position by the elastic member (419). At this time, the insertion portion (116) and needle (450) are inserted into the skin of a user, and the sensor unit (100) from which the protective sheet (160) is separated is attached to the base unit (200) by the adhesive layer (155), so that the body attachable unit (20) is assembled.

After the plunger (410) moves and the insertion portion (116) and the needle (450) are inserted into the skin of a user, the carrier (422) is disengaged from the plunger (410) and the carrier (422) is moved in a direction away from the skin of a user by the elastic member (481). At this time, the needle (450) comes out of the skin of a user and the body attachable unit (20) remains attached to the skin of a user by the adhesive portion (230).

The body attachable unit (20) attached to the skin of a user and separated from the applicator (30) can measure the biometric information of a user and transmit the measurement information to an external terminal (5) or the like.

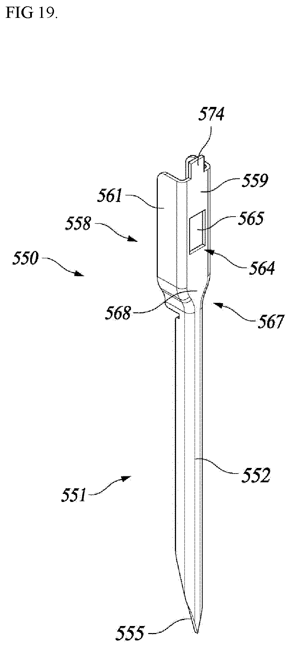

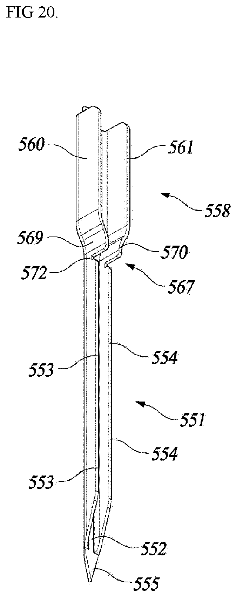

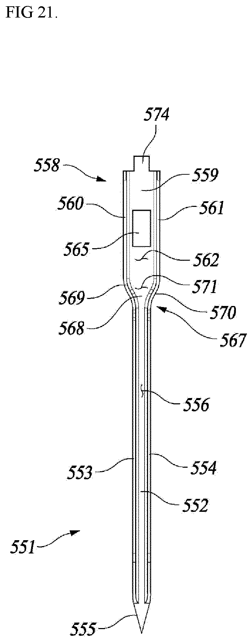

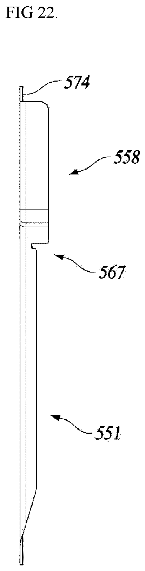

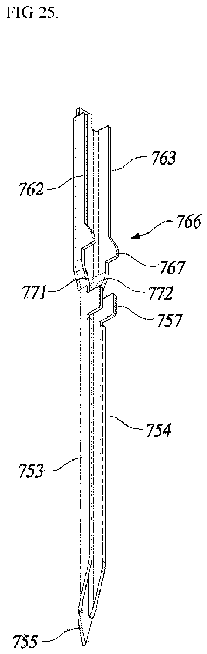

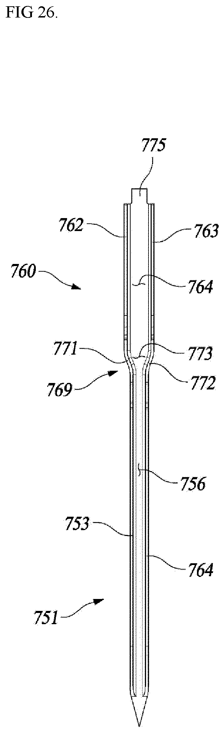

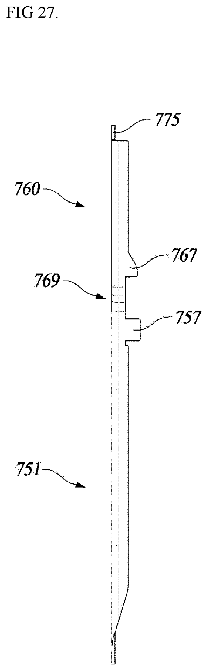

Meanwhile, FIGS. 19 to 29 show various modified examples of needles.

First, the needle (550) shown in FIGS. 19 to 22 includes a needle body (551) inserted into the skin of a user, a needle head (558) coupled to the carrier (422), a needle neck (567) connecting the needle body (551) and the needle head (558), and a needle tail (574) extending from the end of the needle head (558). The needle (550) may be formed as one piece by press-processing the needle body (551), the needle head (558), the needle neck (567), and the needle tail (574) from the same base material.

The needle body (551) includes a needle body base (552) equipped with a needle tip (555) at the end, and a first needle body wing (553) and a second needle body wing (554) disposed on both sides of the needle body base (552). The needle body base (552), the first needle body wing (553), and the second needle body wing (554) form the first channel (556).

The needle head (558) includes a needle head base (559) disposed on the same plane as the needle body base (552), and a first needle head wing (560) and a second needle head wing (561) disposed on both sides of the needle head base (559). The needle head base (559), the first needle head wing (560), and the second needle head wing (561) form the second channel (562). The width of the second channel (562) is larger than the width of the first channel (556). The needle head (558) is provided with a locking portion (564) that engages the clamp portion (427) of the carrier (422). The locking portion (564) includes a needle hole (565) into which the hook (428) of the clamp portion (427) can be inserted.

The needle neck (567) has a shape whose thickness gradually decreases from the needle head (558) toward the needle body (551) and connects the needle body (551) and the needle head (558). The needle neck (567) includes a needle neck base (568) disposed on the same plane as the needle body base (552), a first needle neck wing (569) and a second needle neck wing (570) disposed on both sides of the needle neck base (568). The needle neck base (568), the first needle neck wing (569), and the second needle neck wing (570) form the third channel (571). The third channel (571) has a shape whose width gradually decreases from the second channel (562) toward the first channel (556), and is connected to the first channel (556) and the second channel (562), respectively.

A groove portion (572) is provided between the needle body (551) and the needle neck (567). The groove portion (572) may be used to reduce the weight of the needle (550) and prevent deformation of the needle (550) during the process of forming the needle head (558) from the base material. Additionally, the groove portion (572) may be used for coupling with the carrier (422).

The needle tail (574) extends from the end of the needle head base (559) to be disposed on the same plane as the needle body base (552).

The needle (550) according to this embodiment has the same protrusion height of the needle head wings (560 and 561) protruding from the needle head base (559) and the protrusion height of the needle neck wings (569 and 570) protruding from the needle neck base (568) and has a shape in which the protrusion height of the needle body wings (553 and 554) that protrude from the needle body base (552) is smaller than the protrusion heights of the needle head wings (560 and 561) and the needle neck wings (569 and 570). This type of needle (550) can form a locking portion in which the needle neck (567) engages with the (carrier). And the carrier coupled with the needle (550) according to this embodiment may have a clamp portion in a shape that allows the needle neck (567) to engage.

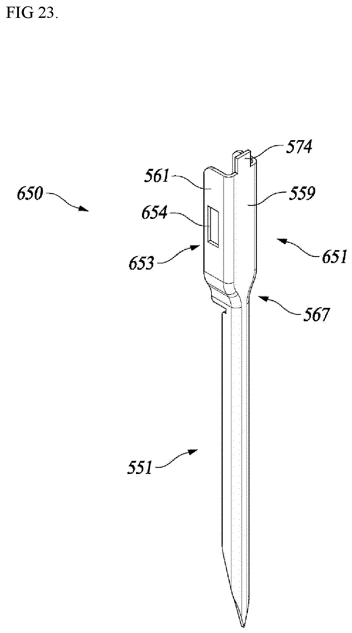

The needle (650) shown in FIG. 23 has a slightly changed configuration of the needle head (651) compared to the needle (550) shown in FIG. 19.

The needle (650) includes a needle body (551) inserted into the skin of a user, a needle head (651) coupled to the carrier (422), a needle neck (567) connecting the needle body (551) and the needle head (651) and a needle tail (574) extending from the end of the needle head (651). The needle (650) may be formed as one piece by press-processing the needle body (551), the needle head (651), the needle neck (567), and the needle tail (574) from the same base material.

The needle head (651) includes a needle head base (559) and a pair of needle head wings (560 and 561) disposed on both sides of the needle head base (559). A pair of needle head wings (560 and 561) is provided with a locking portion (653) that engages with the carrier (422). The locking portion (653) includes a needle hole (654) penetrating the needle head wings (560 and 561).

The carrier coupled to the needle (650) according to this embodiment may include a clamp portion having a hook that is inserted into the needle hole (654).

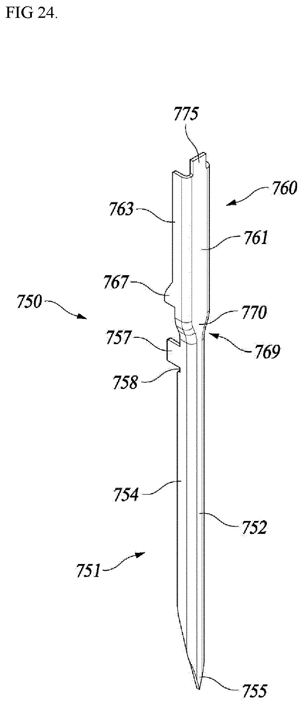

The needle (750) shown in FIGS. 24 to 27 includes a needle body (751) inserted into the skin of a user, a needle head (760) coupled to the carrier (422), a needle neck (769) connecting the needle body (751) and needle head (760) and a needle tail 775 extending from the end of the needle head (760). The needle (750) is formed as one piece by press-processing the needle body (751), the needle head (760), the needle neck (769), and the needle tail (775) from the same base material.

The needle body (751) includes a needle body base (752) equipped with a needle tip (755) at the end, and a first needle body wing (753) and a second needle body wing (754) disposed on both sides of the needle body base (752). The needle body base (752), the first needle body wing (753), and the second needle body wing (754) form the first channel (756). The first needle body wing (753) and the second needle body wing (754) are provided with an extension portion (757) and a groove portion (758). The extension portion (757) extends outward from the end of each of the first needle body wing (753) and the second needle body wing (754). The extension portion (757) may serve to increase the strength of the needle body (751). Additionally, the extension portion (757) may function as a locking portion engaged with the carrier (422). The groove portion (758) may be used for the purpose of reducing the weight of the needle (750) and preventing deformation of the needle body (751) during the process of forming the needle body (751) from the base material. Additionally, the groove portion (758) may be used for coupling with the carrier (422).

The needle head (760) includes a needle head base (761) disposed on the same plane as the needle body base (752), and a first needle head wing (762) and a second needle head wing (763) disposed on both sides of the needle head base (761). The needle head base (761), the first needle head wing (762), and the second needle head wing (763) form the second channel (764). The width of the second channel (764) is larger than the width of the first channel (765).

The carrier coupled with the needle (750) according to the present invention includes a clamp portion into which a hook portion (767) is engaged.

The needle neck (769) has a shape whose thickness gradually decreases from the needle head (760) toward the needle body (751) and connects the needle body (751) and the needle head (760). The needle neck (769) includes a needle neck base (770) disposed on the same plane as the needle body base (752), a first needle neck wing (771), and a second needle neck wing (772) disposed on both sides of the needle neck base (770). The needle neck base (770), the first needle neck wing (771), and the second needle neck wing (772) form the third channel (773). The third channel (773) has a shape whose width gradually decreases from the second channel (764) toward the first channel (756), and is connected to the first channel (756) and the second channel (764), respectively.

The needle tail (775) extends from the end of the needle head base (761) to be disposed on the same plane as the needle head base (761).

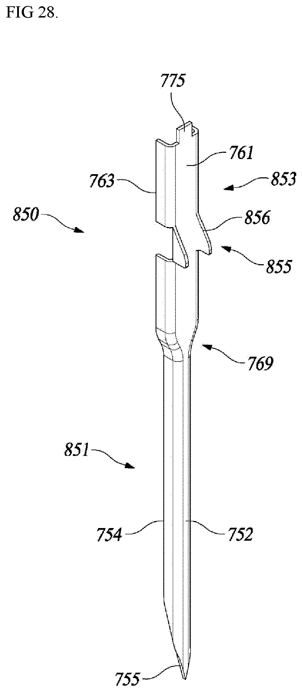

The needle (850) shown in FIG. 28 has a slightly modified configuration of the needle body (851) and the needle head (853) compared to the needle (750) shown in FIG. 24.

The needle (850) includes a needle body (851) inserted into the skin of a user, a needle head (853) coupled to the carrier (422), a needle neck (769) connecting the needle body (851) and the needle head (760) and a needle tail (775) extending from the end of the needle head (853).

The needle body (851) has the extension portion (757) and the groove portion (758) omitted compared to the needle body (751) of the needle (750) described above.

The needle head (853) includes a needle head base (761) and a pair of needle head wings (762 and 763) arranged to face each other on both sides of the needle head base (761). The needle head (853) is provided with a locking portion (855) that can be engaged with the carrier (422). The locking portion (855) includes a hook portion (856) that protrudes from the needle head base (761) in a direction opposite to the direction in which the needle head wings (762 and 763) protrude. The hook portion (856) has a shape in which a middle portion of each of the first needle head wing (762) and the second needle head wing (763) is cut and bent in a direction protruding from the needle head base (761).

The carrier coupled with the needle (850) according to this embodiment may include a clamp portion into which a hook portion (856) may be engaged.

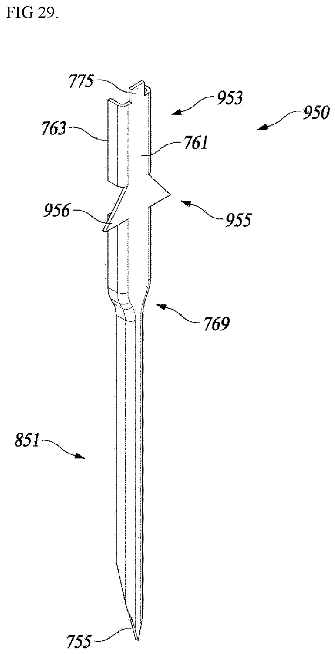

The needle (950) shown in FIG. 29 has a slightly modified needle head (953) configuration compared to the needle (850) shown in FIG. 28.

The needle head (953) includes a needle head base (761) and a pair of needle head wings (762 and 763) arranged to face each other on both sides of the needle head base (761). The needle head (853) is provided with a locking portion (955) that can be engaged with the carrier. The locking portion (955) includes hook portions (956) that protrude from both edges of the needle head base (761).

The carrier coupled to the needle (950) according to this embodiment may include a clamp portion into which a hook portion (956) may be engaged.

In the drawing, the hook portion (956) is shown to lie on the same plane as the needle head base (761), but the shape of the hook portion (956) can be changed in various ways.

In addition, the needle may be formed in various forms in which the needle body, which can be inserted into the skin of a user along with the insertion portion (116) of the sensor (110) through processes such as punching, cutting, and bending of a single base material, and the needle head engaged with the carrier (422) are integrated. As another exemplary embodiment, the needle may be press-processed from the same base material so that the needle body and the needle head are directly connected to each other without a needle neck, so that the needle may take an integrated form. The needle may take an integrated form by being press-processed from the same base material so that the needle body and the needle head are directly connected to each other without a needle neck.

In addition, the drawing shows that the needle body wings of the needle body are bent approximately vertically from the side of the needle body base, but the angle between the needle body base and the needle body wings can be changed in various ways. Likewise, the angle between the needle head base and the needle head wing, or the angle between the needle neck base and the needle neck wing can also be changed in various ways.

In addition, the drawing shows that a stopper that restrains the movement of the moving member is provided in the middle frame in a state in which the elastic member that provides the moving force to the moving member is elastically deformed, but the position of the stopper can be changed in various ways. As another exemplary embodiment, the stopper may be installed at various locations on the applicator body other than the middle frame. As another exemplary embodiment, the stopper can be released by an operating member by being provided on a moving member to engage with the applicator body.

Additionally, as another exemplary embodiment, the base unit may simply take a configuration to support the sensor unit so that it is not separated from the skin of a user. In this case, a separate electronic unit that can process biometric information measured by the sensor unit and transmit it to an external terminal may be detachably coupled to the base unit. A separate electronic unit may be coupled to the base unit to be electrically connected to the sensor unit after the sensor unit is coupled to the base unit. A body attachable unit including such a base unit may be used by coupling a separate electronic unit.

Although the present invention has been shown and described in connection with preferred embodiments for illustrating the invention, the invention is limited to the construction and operation defined in the claims.

## Claims

1. An applicator (30) for inserting a sensor (110) for measuring biometric information into skin of a user, comprising:
an applicator body (300);
a carrier (422) movably mounted to the applicator body (300); and
a needle (750, 850, 950) coupled with the carrier (422) to move with the carrier (422), wherein the needle (750, 850, 950) is configured to be insertable into the skin of the user with the sensor (110), the needle (750, 850, 950) comprising:
a needle body (751, 851) having a first channel (756) accommodating the sensor (110) and to be insertable into the skin of the user;
a needle head (760, 853, 953) having a thickness greater than the needle body (751, 851), wherein the needle body (751, 851) and the needle head (760, 853, 953) are formed as one integrated piece by being press-processed from same base material, wherein the needle head (760, 853, 953) has a locking portion (855, 955) engaged with a clamp portion (427) of the carrier (422), wherein the locking portion (855, 955) is formed as one integrated piece with the needle head (760, 853, 953) and includes a hook portion (767, 856, 956) protruding from the needle head (760, 853, 953), wherein the needle head (760, 853, 953) includes a second channel (764) having a width wider than the first channel (756); and
a needle neck (769) formed as one integrated piece with the needle body (751, 851) and the needle head (760, 853, 953) to connect the needle body (751, 851) and the needle head (760, 853, 953), and having a shape in which a thickness of the needle neck (769) gradually decreases from the needle head (760, 853, 953) toward the needle body (751, 851),
wherein, in the needle neck (769), a third channel (773) is provided to be connected to the first channel (756) and the second channel (764), respectively, and a width of the third channel (773) gradually decreases from the second channel (764) to the first channel (756).

2. The applicator (30) according to claim 1, wherein:
the needle body (751, 851) comprises a needle body base (752) having a needle tip (755) at an end of the needle body base (752),
the needle head (760, 853, 953) comprises a needle head base (761) disposed on a same plane as the needle body base (752), and
the needle neck (769) is formed with a shape of which width gradually decreases from the needle head base (761) toward the needle body base (752) and includes a needle neck base (770) disposed on a same plane as the needle body base (752) and the needle head base (761) to connect the needle body base (752) and the needle head base (761).

3. The applicator (30) according to claim 2, wherein:
the needle body (751, 851) comprises a needle body base (752) having a needle tip (755) at an end of the needle body base (752),
the needle head (760, 853, 953) comprises a needle head base (761) disposed on a same plane as the needle body base (752),
the needle body (751, 851) comprises a first needle body wing (753) and a second needle body wing (754) bent at both sides of the needle body base (752) to form the first channel (756) with the needle body base (752),
the needle head (760, 853, 953) comprises a first needle head wing (762) and a second needle head wing (763) bent at both sides of the needle head base (761) to form the second channel (764) with the needle head base (761), and
the needle neck (769) comprises a first needle neck wing (771) bent at one side of the needle neck base (770) and connecting the first needle body wing (753) and the first needle head wing (762), and a second needle neck wing (772) bent at an other side of the needle neck base (770) and connecting the second needle body wing (754) and the second needle head wing (763), and the first needle neck wing (771) and the second needle neck wing (772) with the needle neck base (770) form a third channel (773) connected to the first channel (756) and the second channel (764), respectively.

4. The applicator (30) according to claim 2, further comprising
a needle tail (775) extended from an end of the needle body base (752) to be disposed on a same plane as the needle body base (752) and having a width narrower than the needle body base (752).

5. The applicator (30) of claim 1, wherein
the needle body (851) comprises a needle body base (752) having a needle tip (755) at an end of the needle body base (752),
the needle head (853) comprises a needle head base (761) disposed on a same plane as the needle body base (752),
the needle body (851) comprises a first needle body wing (753) and a second needle body wing (754) bent at both sides of the needle body base (752) to form the first channel (756) with the needle body base (752),
the needle head (853) comprises a first needle head wing (762) and a second needle head wing (763) bent at both sides of the needle head base (761) to form a second channel (764) with the needle head base (761), and
the hook portion (856) protrudes from the needle head base (761) in a direction opposite to the direction in which the needle head wings (762, 763) protrude.

6. The applicator (30) of claim 1, wherein
the needle body (851) comprises a needle body base (752) having a needle tip (755) at an end of the needle body base (752),
the needle head (853) comprises a needle head base (761) disposed on a same plane as the needle body base (752),
the needle body (851) comprises a first needle body wing (753) and a second needle body wing (754) bent at both sides of the needle body base (752) to form the first channel (756) with the needle body base (752),
the needle head (853) comprises a first needle head wing (762) and a second needle head wing (763) bent at both sides of the needle head base (761) to form a second channel (764) with the needle head base (761), and
the hook portion (956) protrudes from both edges of the needle head base (761).

7. The applicator (30) according to claim 1, wherein:
the carrier (422) has an insertion groove (425) into which the needle head (760, 853, 953) is inserted, and
the clamp portion (427) is disposed in the insertion groove (425).

8. The applicator (30) according to claim 1,
wherein the carrier (422) comprises a carrier body (423) having the clamp portion (427) and a pair of locking arms (439) elastically deformably connected to the carrier body (423) to be detachably engageable with both edges of a sensor housing (120) to which the sensor (110) is mounted.

## Patentansprüche

1. Applikator (30) zum Einführen eines Sensors (110) zum Messen biometrischer Informationen in die Haut eines Benutzers, umfassend:
einen Applikatorkörper (300);
einen Träger (422), der beweglich an dem Applikatorkörper (300) angebracht ist; und
eine Nadel (750, 850, 950), die mit dem Träger (422) gekoppelt ist, um sich mit dem Träger (422) zu bewegen,
wobei die Nadel (750, 850, 950) so ausgebildet ist, dass sie mit dem Sensor (110) in die Haut des Benutzers einführbar ist, wobei die Nadel (750, 850, 950) Folgendes umfasst:
einen Nadelkörper (751, 851), der einen den Sensor (110) aufnehmenden ersten Kanal (756) aufweist und in die Haut des Benutzers einführbar ist;
einen Nadelkopf (760, 853, 953), der eine größere Dicke als der Nadelkörper (751, 851) aufweist, wobei der Nadelkörper (751, 851) und der Nadelkopf (760, 853, 953) durch Pressbearbeitung aus demselben Grundmaterial einstückig ausgebildet sind, wobei der Nadelkopf (760, 853, 953) einen Verriegelungsabschnitt (855, 955) aufweist, der mit einem Klemmabschnitt (427) des Trägers (422) in Eingriff steht, wobei der Verriegelungsabschnitt (855, 955) einstückig mit dem Nadelkopf (760, 853, 953) ausgebildet ist und einen Hakenabschnitt (767, 856, 956) umfasst, der von dem Nadelkopf (760, 853, 953) vorsteht, wobei der Nadelkopf (760, 853, 953) einen zweiten Kanal (764) umfasst, der eine größere Breite als der erste Kanal (756) aufweist; und
einen Nadelhals (769), der einstückig mit dem Nadelkörper (751, 851) und dem Nadelkopf (760, 853, 953) ausgebildet ist, um den Nadelkörper (751, 851) und den Nadelkopf (760, 853, 953) zu verbinden, und eine Form aufweist, bei der eine Dicke des Nadelhalses (769) vom Nadelkopf (760, 853, 953) zum Nadelkörper (751, 851) hin allmählich abnimmt,
wobei in dem Nadelhals (769) ein dritter Kanal (773) vorgesehen ist, um jeweils mit dem ersten Kanal (756) und dem zweiten Kanal (764) verbunden zu sein, und eine Breite des dritten Kanals (773) vom zweiten Kanal (764) zum ersten Kanal (756) allmählich abnimmt.

2. Applikator (30) nach Anspruch 1, wobei:
der Nadelkörper (751, 851) eine Nadelkörperbasis (752) umfasst, die eine Nadelspitze (755) an einem Ende der Nadelkörperbasis (752) aufweist,
der Nadelkopf (760, 853, 953) eine Nadelkopfbasis (761) umfasst, die in derselben Ebene wie die Nadelkörperbasis (752) angeordnet ist, und
der Nadelhals (769) mit einer Form ausgebildet ist, deren Breite von der Nadelkopfbasis (761) zur Nadelkörperbasis (752) hin allmählich abnimmt, und eine Nadelhalsbasis (770) umfasst, die in derselben Ebene wie die Nadelkörperbasis (752) und die Nadelkopfbasis (761) angeordnet ist, um die Nadelkörperbasis (752) und die Nadelkopfbasis (761) zu verbinden.

3. Applikator (30) nach Anspruch 2, wobei:
der Nadelkörper (751, 851) eine Nadelkörperbasis (752) umfasst, die eine Nadelspitze (755) an einem Ende der Nadelkörperbasis (752) aufweist,
der Nadelkopf (760, 853, 953) eine Nadelkopfbasis (761) umfasst, die in derselben Ebene wie die Nadelkörperbasis (752) angeordnet ist,
der Nadelkörper (751, 851) einen ersten Nadelkörperflügel (753) und einen zweiten Nadelkörperflügel (754) umfasst, die an beiden Seiten der Nadelkörperbasis (752) gebogen sind, um mit der Nadelkörperbasis (752) den ersten Kanal (756) zu bilden,
der Nadelkopf (760, 853, 953) einen ersten Nadelkopfflügel (762) und einen zweiten Nadelkopfflügel (763) umfasst, die an beiden Seiten der Nadelkopfbasis (761) gebogen sind, um mit der Nadelkopfbasis (761) den zweiten Kanal (764) zu bilden, und
der Nadelhals (769) einen ersten Nadelhalsflügel (771), der an einer Seite der Nadelhalsbasis (770) gebogen ist und den ersten Nadelkörperflügel (753) und den ersten Nadelkopfflügel (762) verbindet, und einen zweiten Nadelhalsflügel (772) umfasst, der an einer anderen Seite der Nadelhalsbasis (770) gebogen ist und den zweiten Nadelkörperflügel (754) und den zweiten Nadelkopfflügel (763) verbindet, und der erste Nadelhalsflügel (771) und der zweite Nadelhalsflügel (772) mit der Nadelhalsbasis (770) einen dritten Kanal (773) bilden, der jeweils mit dem ersten Kanal (756) und dem zweiten Kanal (764) verbunden ist.

4. Applikator (30) nach Anspruch 2, ferner umfassend
einen Nadelschwanz (775), der sich von einem Ende der Nadelkörperbasis (752) erstreckt, um in derselben Ebene wie die Nadelkörperbasis (752) angeordnet zu sein, und eine geringere Breite als die Nadelkörperbasis (752) aufweist.

5. Applikator (30) nach Anspruch 1, wobei
der Nadelkörper (851) eine Nadelkörperbasis (752) umfasst, die eine Nadelspitze (755) an einem Ende der Nadelkörperbasis (752) aufweist,
der Nadelkopf (853) eine Nadelkopfbasis (761) umfasst, die in derselben Ebene wie die Nadelkörperbasis (752) angeordnet ist,
der Nadelkörper (851) einen ersten Nadelkörperflügel (753) und einen zweiten Nadelkörperflügel (754) umfasst, die an beiden Seiten der Nadelkörperbasis (752) gebogen sind, um mit der Nadelkörperbasis (752) den ersten Kanal (756) zu bilden,
der Nadelkopf (853) einen ersten Nadelkopfflügel (762) und einen zweiten Nadelkopfflügel (763) umfasst, die an beiden Seiten der Nadelkopfbasis (761) gebogen sind, um mit der Nadelkopfbasis (761) einen zweiten Kanal (764) zu bilden, und
der Hakenabschnitt (856) von der Nadelkopfbasis (761) in einer Richtung vorsteht, die der Richtung entgegengesetzt ist, in der die Nadelkopfflügel (762, 763) vorstehen.

6. Applikator (30) nach Anspruch 1, wobei
der Nadelkörper (851) eine Nadelkörperbasis (752) umfasst, die eine Nadelspitze (755) an einem Ende der Nadelkörperbasis (752) aufweist,
der Nadelkopf (853) eine Nadelkopfbasis (761) umfasst, die in derselben Ebene wie die Nadelkörperbasis (752) angeordnet ist,
der Nadelkörper (851) einen ersten Nadelkörperflügel (753) und einen zweiten Nadelkörperflügel (754) umfasst, die an beiden Seiten der Nadelkörperbasis (752) gebogen sind, um mit der Nadelkörperbasis (752) den ersten Kanal (756) zu bilden,
der Nadelkopf (853) einen ersten Nadelkopfflügel (762) und einen zweiten Nadelkopfflügel (763) umfasst, die an beiden Seiten der Nadelkopfbasis (761) gebogen sind, um mit der Nadelkopfbasis (761) einen zweiten Kanal (764) zu bilden, und
der Hakenabschnitt (956) von beiden Rändern der Nadelkopfbasis (761) vorsteht.

7. Applikator (30) nach Anspruch 1, wobei:
der Träger (422) eine Einführnut (425) aufweist, in die der Nadelkopf (760, 853, 953) eingeführt ist, und
der Klemmabschnitt (427) in der Einführnut (425) angeordnet ist.

8. Applikator (30) nach Anspruch 1,
wobei der Träger (422) einen Trägerkörper (423), der den Klemmabschnitt (427) aufweist, und ein Paar Verriegelungsarme (439) umfasst, die elastisch verformbar mit dem Trägerkörper (423) verbunden sind, um mit beiden Rändern eines Sensorgehäuses (120), an dem der Sensor (110) angebracht ist, lösbar in Eingriff gebracht werden zu können.

## Revendications

1. Applicateur (30) pour l'insertion d'un capteur (110) dans la peau d'un utilisateur afin de mesurer des informations biométriques, comprenant :
un corps d'applicateur (300) ;
un support (422) monté de manière mobile sur le corps d'applicateur (300) ; et
une aiguille (750, 850, 950) raccordée au support (422) de manière à être déplaçable avec le support (422), ladite aiguille (750, 850, 950) étant prévue pour être insérable dans la peau de l'utilisateur avec le capteur (110), ladite aiguille (750, 850, 950) comprenant :
un corps d'aiguille (751, 851) comportant un premier canal (756) recevant le capteur (110) et destiné à être inséré dans la peau de l'utilisateur ;
une tête d'aiguille (760, 853, 953) ayant une épaisseur supérieure à celle du corps d'aiguille (751, 851), le corps d'aiguille (751, 851) et la tête d'aiguille (760, 853, 953) étant formés d'un seul tenant par traitement de pressage à partir du même matériau de base, la tête d'aiguille (760, 853, 953) présentant une partie de verrouillage (855, 955) en prise avec une partie de serrage (427) du support (422), la partie de verrouillage (855, 955) étant formée d'un seul tenant avec la tête d'aiguille (760, 853, 953) et comprenant une partie de crochet (767, 856, 956) faisant saillie de la tête d'aiguille (760, 853, 953), la tête d'aiguille (760, 853, 953) comportant un deuxième canal (764) de largeur supérieure à celle du premier canal (756) ; et
un col d'aiguille (769) formé d'un seule tenant avec le corps d'aiguille (751, 851) et la tête d'aiguille (760, 853, 953) de manière à raccorder le corps d'aiguille (751, 851) à la tête d'aiguille (760, 853, 953), et ayant une forme où l'épaisseur du col d'aiguille (769) diminue progressivement de la tête d'aiguille (760, 853, 953) au corps d'aiguille (751, 851),
où, dans le col d'aiguille (769), un troisième canal (773) est prévu pour être raccordé au premier canal (756) et au deuxième canal (764), respectivement, et une largeur du troisième canal (773) diminue progressivement du deuxième canal (764) au premier canal (756).

2. Applicateur (30) selon la revendication 1, où :
le corps d'aiguille (751, 851) comprend une base de corps d'aiguille (752) présentant une pointe d'aiguille (755) à une extrémité de la base de corps d'aiguille (752),
la tête d'aiguille (760, 853, 953) comprend une base de tête d'aiguille (761) disposée sur le même plan que la base de corps d'aiguille (752), et
le col d'aiguille (769) présente une forme dont la largeur diminue progressivement de la base de tête d'aiguille (761) vers la base de corps d'aiguille (752), et comporte une base de col d'aiguille (770) disposée sur le même plan que la base de corps d'aiguille (752) et la base de tête d'aiguille (761) de manière à raccorder la base de corps d'aiguille (752) à la base de tête d'aiguille (761).

3. Applicateur (30) selon la revendication 2, où :
le corps d'aiguille (751, 851) comprend une base de corps d'aiguille (752) ayant une pointe d'aiguille (755) à une extrémité de la base de corps d'aiguille (752),
la tête d'aiguille (760, 853, 953) comprend une base de tête d'aiguille (761) disposée sur le même plan que la base de corps d'aiguille (752),
le corps d'aiguille (751, 851) comprend une première ailette de corps d'aiguille (753) et une deuxième ailette de corps d'aiguille (754) pliées sur les deux côtés de la base de corps d'aiguille (752) de manière à former le premier canal (756) avec la base de corps d'aiguille (752),
la tête d'aiguille (760, 853, 953) comprend une première ailette de tête d'aiguille (762) et une deuxième ailette de tête d'aiguille (763) pliée sur les deux côtés de la base de tête d'aiguille (761) de manière à former le deuxième canal (764) avec la base de tête d'aiguille (761), et
le col d'aiguille (769) comprend une première ailette de col d'aiguille (771) pliée sur un côté de la base de col d'aiguille (770) et raccordant la première ailette de corps d'aiguille (753) à la première ailette de tête d'aiguille (762), et une deuxième ailette de col d'aiguille (772) pliée sur un autre côté de la base de col d'aiguille (770) et raccordant la deuxième ailette de corps d'aiguille (754) à la deuxième ailette de tête d'aiguille (763), et où la première ailette de col d'aiguille (771) et la deuxième ailette de col d'aiguille (772) avec la base de col d'aiguille (770) forment un troisième canal (773) raccordé au premier canal (756) et au deuxième canal (764), respectivement.

4. Applicateur (30) selon la revendication 2, comprenant en outre un empennage d'aiguille (775) s'étendant à partir d'une extrémité de la base de corps d'aiguille (752) de manière à être disposé sur le même plan que la base de corps d'aiguille (752) et ayant une largeur inférieure à celle de la base de corps d'aiguille (752).

5. Applicateur (30) selon la revendication 1, où
le corps d'aiguille (851) comprend une base de corps d'aiguille (752) présentant une pointe d'aiguille (755) à une extrémité de la base de corps d'aiguille (752),
la tête d'aiguille (853) comprend une base de tête d'aiguille (761) disposée sur le même plan que la base de corps d'aiguille (752),
le corps d'aiguille (851) comprend une première ailette de corps d'aiguille (753) et une deuxième ailette de corps d'aiguille (754) pliées sur les deux côtés de la base de corps d'aiguille (752) de manière à former le premier canal (756) avec la base de corps d'aiguille (752),
la tête d'aiguille (853) comprend une première ailette de tête d'aiguille (762) et une deuxième ailette de tête d'aiguille (763) pliées sur les deux côtés de la base de tête d'aiguille (761) de manière à former un deuxième canal (764) avec la base de tête d'aiguille (761), et
la partie de crochet (856) fait saillie de la base de tête d'aiguille (761) dans une direction opposée à la direction de saillie des ailettes de tête d'aiguille (762, 763).

6. Applicateur (30) selon la revendication 1, où
le corps d'aiguille (851) comprend une base de corps d'aiguille (752) présentant une pointe d'aiguille (755) à une extrémité de la base de corps d'aiguille (752),
la tête d'aiguille (853) comprend une base de tête d'aiguille (761) disposée sur le même plan que la base de corps d'aiguille (752),
le corps d'aiguille (851) comprend une première ailette de corps d'aiguille (753) et une deuxième ailette de corps d'aiguille (754) pliées sur les deux côtés de la base de corps d'aiguille (752) de manière à former le premier canal (756) avec la base de corps d'aiguille (752),
la tête d'aiguille (853) comprend une première ailette de tête d'aiguille (762) et une deuxième ailette de tête d'aiguille (763) pliées sur les deux côtés de la base de tête d'aiguille (761) de manière à former un deuxième canal (764) avec la base de tête d'aiguille (761), et
la partie de crochet (956) fait saillie des deux bords de la base de tête d'aiguille (761).

7. Applicateur (30) selon la revendication 1, où :
le support (422) comporte une rainure d'insertion (425) dans laquelle la tête d'aiguille (760, 853, 953) est insérée, et
la partie de serrage (427) est disposée dans la rainure d'insertion (425).

8. Applicateur (30) selon la revendication 1,
où le support (422) comprend un corps de support (423) pourvu de la partie de serrage (427) et une paire de bras de verrouillage (439) raccordés de manière élastiquement déformable au corps de support (423) afin d'être en prise de manière amovible avec les deux bords d'un boîtier de capteur (120) sur lequel est monté le capteur (110).
